# EUROPEAN PATENT APPLICATION

(11) **EP 4 497 826 A1**
(43) Date of publication of application: **29.01.2025**
(21) Application number: 23791960.0
(22) Date of filing: 21.04.2023
(51) Int. Cl.: C12N 15/12, A61K 38/17, A61K 48/00, A61P 21/00, A61P 25/00, A61P 43/00, C07K 14/46

(54) **TDP-43 AGGREGATION SUPPRESSING AGENT AND PHARMACEUTICAL COMPOSITION**

(30) Priority: 22.04.2022 JP 2022070532
(71) Applicant: Niigata University, Niigata-shi, Niigata 950-2181 (JP)
(72) Inventor: SUGAI Akihiro, Niigata-shi, Niigata 951-8585 (JP); YAMAGISHI Takuma, Niigata-shi, Niigata 951-8510 (JP); YAMADA Yumi, Niigata-shi, Niigata 951-8510 (JP); ONODERA Osamu, Niigata-shi, Niigata 951-8585 (JP)
(74) Representative: Cabinet Becker et Associés
(86) International application number: PCT/JP2023/016000
(87) International publication number: WO 2023/204313

(57) **Abstract**

An aggregation inhibitor of TDP-43 which specifically acts on TDP-43 and has an aggregation inhibitory effect on TDP-43 without affecting the expression level of TDP-43 itself, and a pharmaceutical composition formed using the aggregation inhibitor of TDP-43 are provided. The aggregation inhibitor of TDP-43 contains a modified TDP-43 protein which contains at least an N-terminus domain and does not contain an intrinsically disordered region at the C-terminus, or a nucleic acid encoding the modified TDP-43 protein. The pharmaceutical composition contains the aggregation inhibitor of TDP-43 as an active ingredient, and is used at preventing or treating TDP-43 proteinopathy.

## Description

### TECHNICAL FIELD

The present invention relates to an aggregation inhibitor of TDP-43 and a pharmaceutical composition.

Priority is claimed on Japanese Patent Application No. 2022-070532, filed April 22, 2022, the content of which is incorporated herein by reference.

### BACKGROUND ART

A neurological disorder in which TAR DNA binding protein 43 (TDP-43) aggregates and accumulates is collectively referred to as TDP-43 proteinopathy. TDP-43 proteinopathy includes amyotrophic lateral sclerosis (ALS) and frontotemporal lobar degeneration (FTLD). However, no fundamental treatment method has been found for any of these diseases. In these diseases, the disappearance of the intranuclear protein TDP-43 from the nucleus and the aggregation of the intranuclear protein TDP-43 in the cytoplasm are deeply involved in the pathological state.

As a drug targeting TDP-43, an antisense nucleic acid that induces degradation of TDP-43 mRNA (for example, refer to Patent Document 1 and the like) or an antibody that binds to a TDP-43 protein has been proposed (for example, refer to Patent Documents 2 to 8 and the like), but these are not associated with a specific therapeutic drug.

An intrinsically disordered region (IDR) of TDP-43 is a flexible region that does not have a specific structure, and is involved in the formation of liquid-liquid phase separation that interacts with various molecules to form organelles such as RNA granules without a membrane. There is accumulated evidence that the abnormality or persistence of the liquid-liquid phase separation is involved in the functional abnormality or aggregation of TDP-43. The IDR of TDP-43 is also referred to as a prion-like domain, and is a region that is a core of aggregation. The IDR code region of TDP-43 is completely included in the selectively spliced intron 6. Intron 6, together with intron 7, is spliced in an intranuclear TDP-43 amount-dependent manner, thereby contributing to the autoregulation of the expression of TDP-43. Therefore, these splicings are reduced in a pathological state in which the intranuclear TDP-43 is reduced (for example, refer to

Non-Patent Documents 1, 2, and the like). In addition, the function of TDP-43 autoregulation involved in the splicing is also reduced by some of the ALS-causing mutations (for example, refer to Non-Patent Documents 3, 4, and the like). On the contrary, in the case where splicing of the intron 6 is promoted, TDP-43 lacking IDR is expressed. In human motor neurons, a hypothesis has been proposed based on a high expression ratio of mRNA encoding TDP-43 lacking an IDR being observed, whereas TDP-43 lacking an excessive IDR exhibits toxicity (for example, refer to Non-Patent Document 5 and the like).

### Citation List

### Patent Documents

Patent Document 1: PCT International Publication No. WO 2019/013141
Patent Document 2: PCT International Publication No. WO 2009/008529
Patent Document 3: PCT International Publication No. WO 2013/061163
Patent Document 4: PCT International Publication No. WO 2015/117088
Patent Document 5: PCT International Publication No. WO 2016/053610
Patent Document 6: PCT International Publication No. WO 2019/134981
Patent Document 7: PCT International Publication No. WO 2019/177138
Patent Document 8: PCT International Publication No. WO 2020/118458

### Non-Patent Documents

Non-Patent Document 1: Koyama, A. et al., "Increased cytoplasmic TARDBP mRNA in affected spinal motor neurons in ALS caused by abnormal autoregulation of TDP-43.", Nucleic Acids Res., Vol. 44, pp. 5820-5836, 2016.

Non-Patent Document 2: Liu EY et al., "Loss of Nuclear TDP-43 is Associated with Decondensation of LINE Retrotransposons.", Cell Rep., Vol. 27, Issue 5, pp. 1409-1421.e6., 2019.

Non-Patent Document 3: White MA et al., "TDP-43 gains function due to perturbed autoregulation in a Tardbp knock-in mouse model of ALS-FTD.", NatNeurosci., Vol. 21, Issue 4, pp. 552-563, 2018.

Non-Patent Document 4: Hallegger M, et al., "TDP-43 condensation properties specify its RNA-binding and regulatory repertoire", Cell, Vol. 184, Issue 18, pp. 4680-4696, 2021.

Non-Patent Document 5: Weskamp K et al., "Shortened TDP43 isoforms upregulated by neuronal hyperactivity drive TDP43 pathology in ALS.", J Clin Invest., Vol. 130, Issue 3, pp. 1139-1155, 2020.

### SUMMARY OF INVENTION

### Technical Problem

In order to fundamentally overcome TDP-43 proteinopathy, the development of a therapeutic drug that directly targets TDP-43 is required. However, the antisense nucleic acid that degrades TDP-43 mRNAto inhibit the expression described in Patent Document 1 and the like causes a loss of function due to a decrease in intranuclear TDP-43, and thus is not suitable as a therapeutic drug. In addition, there is similar concern regarding an antibody drug that leads to degradation by targeting TDP-43 protein described in Patent Documents 2 to 8 and the like, and there is also concern regarding safety since the antibody drug is an exogenous molecule.

In order to solve these problems, it is necessary to find a molecule that specifically acts on TDP-43 and has an effect of inhibiting the aggregation of TDP-43 without affecting the expression level of TDP-43 itself.

The present invention has been made in view of the above circumstances, and an object thereof is to provide an aggregation inhibitor of TDP-43 which specifically acts on TDP-43 and has an aggregation inhibitory effect on TDP-43 without affecting the expression level of TDP-43 itself, and a pharmaceutical composition formed using the aggregation inhibitor of TDP-43.

### Solution to Problem

As a result of intensive studies to achieve the above object, the inventors of the present invention have focused on the fact that a TDP-43 isoform in which an IDR most involved in aggregation property is completely removed is generated by splicing intron 6 of pre-mRNA of TDP-43, as an in vivo molecule that has the possibility of inhibiting aggregation of TDP-43, and have found that the TDP-43 isoform lacking the IDR binds to TDP-43 through an N-terminus domain (NTD) to inhibit the aggregation of TDP-43, thereby completing the present invention.

That is, the present invention includes the following aspects.
(1) An aggregation inhibitor of TDP-43, including: a modified TDP-43 protein which contains at least an N-terminus domain and does not contain an intrinsically disordered region at the C-terminus, or a nucleic acid encoding the modified TDP-43 protein.
(2) The aggregation inhibitor of TDP-43 according to (1), further including: a protein consisting of an amino acid sequence which contains at least amino acid residues from the 1st to the 77th from an N-terminus and does not contain amino acid residues from the 278th to the 414th from the N-terminus in the amino acid sequence represented by SEQ ID NO: 1, or a nucleic acid encoding the protein.
(3) The aggregation inhibitor of TDP-43 according to (1) or (2), further including: a protein consisting of an amino acid sequence represented by SEQ ID NO: 2 or 3, or a nucleic acid encoding the protein.
(4) The aggregation inhibitor of TDP-43 according to any one of (1) to (3), further including: a nucleic acid consisting of a base sequence represented by SEQ ID NO: 4 or 5.
(5) The aggregation inhibitor of TDP-43 according to any one of (1) to (4), in which a 3' untranslated region including a TDP-43 binding motif is added to the nucleic acid.
(6) The aggregation inhibitor of TDP-43 according to (5), in which the TDP-43 binding motif consists of a sequence in which thymine and guanine are alternately repeated three or more times.
(7) The aggregation inhibitor of TDP-43 according to (6), further including: a nucleic acid consisting of a base sequence represented by SEQ ID NO: 6.
(8) A pharmaceutical composition including: the aggregation inhibitor of TDP-43 according to any one of (1) to (7) as an active ingredient, in which the pharmaceutical composition is used at preventing or treating TDP-43 proteinopathy.
(9) The pharmaceutical composition according to (8), in which TDP-43 proteinopathy is frontotemporal lobar degeneration or amyotrophic lateral sclerosis. Advantageous Effects of Invention

According to the aggregation inhibitor of TDP-43 of the above-described aspect, it is possible to inhibit the aggregation of TDP-43 by acting specifically on TDP-43 without affecting the expression level of TDP-43 itself. The pharmaceutical composition according to the above aspect contains the aggregation inhibitor of TDP-43, and can prevent or treat TDP-43 proteinopathy.

### BRIEF DESCRIPTION OF DRAWINGS

[FIG. 1] A conceptual diagram showing an aspect of aggregation inhibition by a splicing variant of TDP-43 lacking an IDR.
[FIG. 2A] A schematic view showing TDP-43 and a modified body of TDP-43 used in Examples.
[FIG. 2B] A differential interference microscope image in Example 1.
[FIG. 3A] A graph showing the aggregation property of TDP-43, ΔNTD-sTDP, and a mixed protein thereof in Example 2.
[FIG. 3B] A graph showing the aggregation property of TDP-43 and a mixed protein of ΔNTD-sTDP, sTDP (intron 6 splicing), or sTDP (to exon 5) in Example 2.
[FIG. 4A] A graph showing the stability of TDP-43 and sTDP (intron 6 splicing) in Example 3.
[FIG. 4B] A graph showing the stability of TDP-43 under sTDP (intron 6 splicing) or ΔNTD-sTDP expression in Example 3.
[FIG. 5] A graph showing the retention rate of exon 18 of Sort1 mRNA in a ventricle of a TDP-43 transgenic mouse in Example 4.
[FIG. 6A] A graph (upper part) showing the position and strength of the TDP-43 binding motif in the 3' UTR of a wild-type or modified sTDP (intron 6 splicing) using RBPmap in Example 5, and a schematic configuration diagram (lower part) of the 3' UTR of the wild-type or modified sTDP (intron 6 splicing).
[FIG. 6B] An image (left) showing the result of Western blotting analysis using an anti-TDP-43 antibody in Example 5, and a graph (right) in which the expression level of a wild-type or modified sTDP (intron 6 splicing) is quantified from a band of the results of the Western blotting analysis.
[FIG. 7A] A schematic view showing the verification system in Example 6.
[FIG. 7B] A photographic view of the fluorescence of each system in Example 6 observed with a microscope.
[FIG. 7C] A graph showing the proportion of cells in which aggregates are recognized in each system in Example 6.
[FIG. 8A] A schematic view showing the verification system in Example 7.
[FIG. 8B] A photographic view of the fluorescence of each system in Example 7 observed with a microscope.
[FIG. 8C] A graph showing the distribution of a correlation of the fluorescence intensity of the modified UTR in Example 7.
[FIG. 8D] A graph showing the distribution of a correlation between fluorescence intensities of siCtrl and the modified UTR of siTDP43 in Example 7.
[FIG. 8E] A view showing the sequence of a modified portion of 3'UTR of the modified UTR in Example 7.

### DESCRIPTION OF EMBODIMENTS

Hereinafter, an aggregation inhibitor of TDP-43 and a pharmaceutical composition according to an embodiment of the present invention (hereinafter, simply referred to as "present embodiment") will be described in detail.

### <TDP-43 protein>

The TAR DNA-binding protein-43 (TDP-43) protein is a protein encoded by the TARDBP gene in humans.

The amino acid sequence of the full-length human TDP-43 protein is disclosed in Genbank accession number NP_031401.1.

The base sequence of the full-length mRNA of human TDP-43 is disclosed in Genbank accession number NM_007375.4.

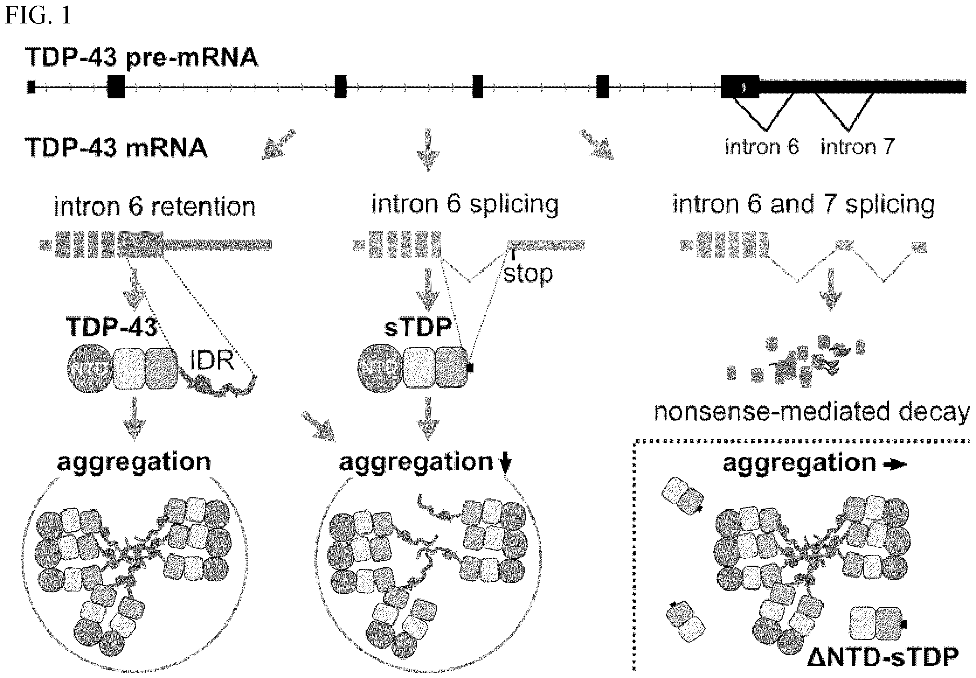

By a combination of selective splicing including the 3'UTR of pre-mRNA of TDP-43 and the use of a selective polyadenylation site, a large number of isoforms (splicing variants) are produced in the mRNA of the TDP-43 gene. Among these splicing variants, the inventors focused on a variant in which the intron 6 of the pre-mRNA of TDP-43 is spliced and the IDR is completely removed, and hypothesized that in the case where this variant lacking the IDR binds to TDP-43 through NTD, the IDR density is inhibited, and as a result, the abnormal liquid-liquid phase separation and aggregation are inhibited. FIG. 1 is a conceptual diagram showing an aspect of aggregation inhibition by a variant lacking an IDR.

As a result of intensive studies to verify the above hypothesis, inventors of the present invention found that the liquid-liquid phase separation and aggregation due to excessive TDP-43 are inhibited by a variant having an NTD and lacking an IDR, as shown in Examples described later, thereby completing the present invention.

### «TDP-43 aggregation inhibitor»

The TDP-43 aggregation inhibitor of the present embodiment contains a modified TDP-43 protein which contains at least an N-terminus domain (NTD) and does not contain an intrinsically disordered region (IDR) at the C-terminus, or a nucleic acid encoding the modified TDP-43 protein.

The aggregation inhibitor of TDP-43 according to the present embodiment acts specifically on TDP-43 and inhibits the aggregation of TDP-43 and the abnormal liquid-liquid phase separation without affecting the expression level of TDP-43 itself.

### <Modified TDP-43 protein>

The modified TDP-43 protein (hereinafter, simply referred to as "modified body" in some cases) contains at least an NTD and does not contain an IDR at the C-terminus.

The modified TDP-43 protein has an NTD, and thus can bind to a wild-type TDP-43 protein having an IDR at the C-terminus through the NTD to form a dimer or an oligomer. On the other hand, since the modified TDP-43 protein does not contain an IDR at the C-terminus, the modified TDP-43 protein can inhibit the level of density of IDR in the wild-type TDP-43 protein having an IDR at the C-terminus.

The modified TDP-43 protein may have the above-described structure, and may be, for example, a modified body consisting of only an NTD, a modified body in which only an IDR is deleted from a wild-type TDP-43 protein, a modified body not consisting of only an NTD, in which at least an IDR is deleted from a wild-type TDP-43 protein, or a modified body in which an amino acid sequence completely unrelated to the amino acid sequence of the TDP-43 protein binds to the NTD. Among these, since the modified TDP-43 protein is a deletion body based on TDP-43 present as an in vivo molecule, a modified body consisting of only an NTD, a modified body in which only an IDR is deleted from a wild-type TDP-43 protein, or a modified body not consisting of only an NTD, in which at least an IDR is deleted from a wild-type TDP-43 protein, is preferable, and since the modified TDP-43 protein has a peptide length sufficient to inhibit the level of density of an IDR, a modified body in which only an IDR is deleted from a wild-type TDP-43 protein, or a modified body not consisting of only an NTD, in which at least an IDR is deleted from a wild-type TDP-43 protein, is more preferable.

Specific examples of such a modified body include a protein consisting of an amino acid sequence which contains at least amino acid residues from the 1st to the 77th from the N-terminus and does not contain amino acid residues from the 278th to the 414th from the N-terminus, in the amino acid sequence represented by SEQ ID NO: 1.

SEQ ID NO: 1 is a full-length amino acid sequence of the wild-type TDP-43 protein consisting of 414 amino acid residues (Genbank accession number NP_031401.1). In addition, the amino acid residues from the 1st to the 77th from the N-terminus are NTD, and the amino acid residues from the 278th to the 414th from the N-terminus are IDR, in the amino acid sequence represented by SEQ ID NO: 1.

Examples of the preferable modified body include the following, but are not limited thereto.

A modified body consisting of amino acid residues from the 1st to the 77th from the N-terminus in an amino acid sequence represented by SEQ ID NO: 1;
a modified body consisting of amino acid residues from the 1st to the 79th from the N-terminus (modified body consisting of a protein encoded by exon 2 of TDP-43 mRNA) in an amino acid sequence represented by SEQ ID NO: 1;
a modified body consisting of amino acid residues from the 1st to the 134th from the N-terminus (modified body consisting of a protein encoded by exons 2 and 3 of TDP-43 mRNA) in an amino acid sequence represented by SEQ ID NO: 1;
a modified body consisting of amino acid residues from the 1st to the 181st from the N-terminus (modified body consisting of a protein encoded by exons 2 to 4 of TDP-43) in an amino acid sequence represented by SEQ ID NO: 1;
a modified body consisting of amino acid residues from the 1st to the 238th from the N-terminus (modified body consisting of a protein encoded by exons 2 to 5 of TDP-43) in an amino acid sequence represented by SEQ ID NO: 1;
a modified body in which only amino acid residues from the 278th to the 414th from the N-terminus are deleted (modified body encoded by TDP-43 mRNA spliced with intron 6 and added with a sequence after splicing) in an amino acid sequence represented by SEQ ID NO: 1.

Alternatively, the above-described modified body is one modified body in which an amino acid is deleted from the C-terminus of the TDP-43 protein, but for example, a modified body in which the inside of the amino acid sequence of the TDP-43 protein is deleted is also preferably an exemplary example as follows.

A modified body consisting of proteins encoded by exons 2 and 4 of the TDP-43 mRNA;
a modified body consisting of proteins encoded by exon 2 and 5 of the TDP-43 mRNA;
a modified body consisting of proteins encoded by exons 2, 3, and 5 of the TDP-43 mRNA;
a modified body consisting of proteins encoded by exons 2, 4, and 5 of the TDP-43 mRNA.

In addition, as the modified body, a protein which consists of an amino acid sequence of the following (a1) or (a2) and has an aggregation inhibitory action of TDP-43 can also be used.
(a1) An amino acid sequence which contains amino acid residues from the 1st to the 77th from the N-terminus, and does not contain amino acid residues from the 278th to the 414th from the N-terminus in the amino acid sequence represented by SEQ ID NO: 1, and has an identity of 80% or more and less than 100%, preferably 85% or more and less than 100%, more preferably 90% or more and less than 100%, further more preferably 95% or more and less than 100%, more preferably 97% or more and less than 100%, further more preferably 98% or more and less than 100%, and particularly preferably 99% or more and less than 100% with an amino acid sequence obtained by removing the amino acid residues from the 1st to the 77th from the N-terminus;
(a2) an amino acid sequence which contains amino acid residues from the 1st to the 77th from the N-terminus, does not contain amino acid residues from the 278th to the 414th from the N-terminus, in the amino acid sequence represented by SEQ ID NO: 1, and in which one or several amino acids are deleted, substituted, or added in the amino acid sequence obtained by removing the amino acid residues from the 1st to the 77th from the N-terminus.

In the present specification, the sequence identity of an amino acid sequence is a value indicating a proportion at which an amino acid sequence of a subject (subject amino acid sequence) is matched with respect to an amino acid sequence of a reference amino acid sequence (reference amino acid sequence). The sequence identity of the subject amino acid sequence with respect to the reference amino acid sequence can be determined, for example, as follows.

First, the reference amino acid sequence and the subject amino acid sequence are aligned. Each amino acid sequence may include gaps to maximize the sequence identity. Subsequently, the number of matched amino acids in the reference amino acid sequence and the subject amino acid sequence can be calculated, and the sequence identity can be obtained according to the following formula. Sequence identity (%) of amino acid sequence = number of matched amino acids/total number of amino acids of subject amino acid sequence × 100

In addition, the number of amino acids that may be deleted, substituted, or added is preferably 1 or more and 55 or less, more preferably 1 or more and 40 or less, further more preferably 1 or more and 27 or less, more preferably 1 or more and 13 or less, further more preferably 1 or more and 8 or less, particularly preferably 1 or more and 5 or less, and most preferably 1 or more and 2 or less.

Among these, in the amino acid sequence represented by SEQ ID NO: 1, a modified body in which only amino acid residues from the 278th to the 414th from the N-terminus are deleted (modified body encoded by TDP-43 mRNA spliced with intron 6 and added with a sequence after splicing) or a modified body consisting of amino acid residues from the 1st to the 238th from the N-terminus (modified body consisting of proteins encoded by exons 2 to 5 of TDP-43), that is, a protein consisting ofan amino acid sequence represented by SEQ ID NO: 2 or 3 is preferable.

The modified TDP-43 protein may be produced by a known chemical synthesis method, or may be produced by a known biological method, but is preferably produced by a biological method. Examples of the biological method include a method using a cell-free protein synthesis system or a living cell protein synthesis system with an expression vector having a nucleic acid encoding the modified TDP-43 protein.

The expression of the modified TDP-43 protein using a cell-free peptide synthesis system may be carried out by appropriately using, for example, a commercially available kit such as Premium Expression Kit (manufactured by Cell Free Sciences, Inc.) containing a wheat germ extract, RTS 100, E. coli HY Kit containing an E. coli extract (manufactured by Roche Applied Science), cell-free Quick (manufactured by Taiyo Nippon Sanso Corporation), or the like. In the case where the expressed modified TDP-43 protein is insoluble, the protein may be appropriately solubilized using a protein denaturant such as guanidine hydrochloride and urea. The modified TDP-43 protein can also be prepared by a fractionation treatment by a fractional centrifugation method, a sucrose density gradient centrifugation method, or the like, or a purification method using an affinity column, ion-exchange chromatography, or the like.

A method for producing a modified TDP-43 protein using a living cell peptide synthesis system can be performed by introducing a vector containing a nucleic acid encoding a modified TDP-43 protein into a living cell and culturing the living cell in a culture solution containing nutrients, antibiotics, and the like. The living cell is not particularly limited as long as it is a living cell capable of expressing a vector containing a nucleic acid encoding the modified TDP-43 protein, and examples thereof include a mammalian cell line such as Chinese hamster ovary (CHO) cells or a living cell such as Escherichia coli, yeast cells, insect cells, and plant cells, and in consideration of simplicity and cost-effectiveness, Escherichia coli is preferable. The expression of a vector containing the modified TDP-43 protein can be carried out by incorporating the modified TDP-43 protein into an expression vector designed to be expressed in each living cell by a gene recombination technology and introducing the expression vector into the living cell. In addition, the introduction of the vector containing a nucleic acid encoding the modified TDP-43 protein into the living cell can be carried out by a suitable method for the living cell. As such a method, for example, an electroporation method, a heat-shock method, a calcium phosphate method, a lipofection method, a DEAE-dextran method, a microinjection method, a particle gun method, or a method using a virus and the like can be selected. In addition, examples thereof include a method using a commercially available transfection reagent such as FuGENE (registered trademark) 6 Transfection Reagent (manufactured by Roche), Lipofectamine 2000 Reagent (manufactured by Thermo Fisher Scientific, Inc.), Lipofectamine LTX Reagent (manufactured by Thermo Fisher Scientific, Inc.), and Lipofectamine 3000 Reagent (manufactured by Thermo Fisher Scientific, Inc.).

The modified TDP-43 protein expressed by the living cell peptide synthesis system can be prepared by subjecting the living cell containing the modified TDP-43 protein to a crushing treatment or an extraction treatment. Examples of the crushing treatment include a physical crushing treatment using a freeze-thaw method, a French press, glass beads, a homogenizer, an ultrasonic crushing device, or the like. In addition, examples of the extraction treatment include an extraction treatment using a protein denaturant such as guanidine hydrochloride and urea. The modified TDP-43 protein can also be prepared by a fractionation treatment by a fractional centrifugation method, a sucrose density gradient centrifugation method, or the like, or a purification method using an affinity column, ion-exchange chromatography, or the like.

### <Nucleic acid encoding modified TDP-43 protein>

The nucleic acid is not particularly limited as long as it encodes the above-described modified TDP-43 protein, and examples thereof include a nucleic acid consisting of a sequence that contains at least consecutive bases from the 112th to the 333rd from the 5' terminus and does not contain consecutive bases from the 935th to the 1344th from the 5' terminus, in the base sequence represented by SEQ ID NO: 7.

The SEQ ID NO: 7 is a full-length base sequence of the wild-type TDP-43 mRNA consisting of 4185 bases (Genbank accession number NM_007375.4). In addition, the consecutive bases from the 112th to the 333rd from the 5' terminus are regions encoding NTD, and the consecutive bases from the 935th to the 1344th from the 5' terminus are regions encoding IDR, in the base sequence represented by SEQ ID NO: 7.

Preferable examples of the nucleic acid include the following, but are not limited thereto.

A nucleic acid consisting of consecutive bases from the 112th to the 333rd from the 5' terminus in the base sequence represented by SEQ ID NO: 7;
a nucleic acid consisting of consecutive bases from the 112th to the 340th from the 5' terminus (nucleic acid consisting of exon 2 of TDP-43 mRNA) in the base sequence represented by SEQ ID NO: 7;
a nucleic acid consisting of consecutive bases from the 112th to the 504th from the 5' terminus (nucleic acid consisting of exons 2 and 3 of TDP-43 mRNA) in the base sequence represented by SEQ ID NO: 7;
a nucleic acid consisting of consecutive bases from the 112th to the 645th from the 5' terminus (nucleic acid consisting of exons 2 to 4 of TDP-43 mRNA) in the base sequence represented by SEQ ID NO: 7;
a nucleic acid consisting of consecutive bases from the 112th to the 816th from the 5' terminus (nucleic acid consisting of exons 2 to 5 of TDP-43 mRNA) in the base sequence represented by SEQ ID NO: 7;
a nucleic acid in which only consecutive bases from the 935th to the 1885th from the 5' terminus are deleted (TDP-43 mRNA spliced with intron 6 and added with a sequence after splicing) in the base sequence represented by SEQ ID NO: 7.

Alternatively, the above-described nucleic acid is one in which a base is deleted from the 3' terminus of TDP-43 mRNA, but for example, a modified body in which the inside of the base sequence of TDP-43 mRNA is deleted is also preferably an exemplary example as follows.

A nucleic acid consisting of exons 2 and 4 of TDP-43 mRNA;
a nucleic acid consisting of exons 2 and 5 of TDP-43 mRNA;
a nucleic acid consisting of exons 2, 3, and 5 of TDP-43 mRNA;
a nucleic acid consisting of exons 2, 4, and 5 of TDP-43 mRNA.

In addition, as the nucleic acid, a nucleic acid consisting of any of the following base sequences (b1) to (b3) and having an aggregation inhibitory action of TDP-43 can also be used.
(b1) A base sequence which contains consecutive bases from the 112th to the 333rd from the 5' terminus, and does not contain consecutive bases from the 935th to the 1344th from the 5' terminus in the base sequence represented by SEQ ID NO: 7, and has an identity of 80% or more and less than 100%, preferably 85% or more and less than 100%, more preferably 90% or more and less than 100%, further more preferably 95% or more and less than 100%, more preferably 97% or more and less than 100%, further more preferably 98% or more and less than 100%, and particularly preferably 99% or more and less than 100% with a base sequence obtained by removing the consecutive bases from the 112th to the 333rd from the 5' terminus;
(b2) a base sequence which contains consecutive bases from the 112th to the 333rd from the 5' terminus, does not contain consecutive bases from the 935th to the 1344th from the 5' terminus, and is obtained by removing consecutive bases from the 112th to the 333rd from the 5' terminus, in which one or several bases are deleted, substituted, or added in the base sequence represented by SEQ ID NO: 7;
(b3) a base sequence which can be hybridized with a nucleic acid consisting of a base sequence complementary to a nucleic acid consisting of a base sequence that contains consecutive bases from the 112th to the 333rd from the 5' terminus and does not contain consecutive bases from the 935th to the 1344th from the 5' terminus in the base sequence represented by SEQ ID NO: 7 under stringent conditions.

In the present specification, the sequence identity of the subject base sequence with respect to the reference base sequence can be obtained, for example, as follows. First, the reference base sequence and the subject base sequence are aligned. Each base sequence may include a gap to maximize the sequence identity. Subsequently, the number of matched bases in the reference base sequence and the subject base sequence can be calculated, and the sequence identity can be obtained according to the following formula. Sequence identity (%) of base sequence = number of matched bases/total number of bases of subject base sequence × 100

In addition, the number of bases that may be deleted, substituted, or added is preferably 1 or more and 700 or less, more preferably 1 or more and 500 or less, further more preferably 1 or more and 300 or less, more preferably 1 or more and 100 or less, more preferably 1 or more and 75 or less, particularly preferably 1 or more and 30 or less, and most preferably 1 or more and 10 or less.

In the present specification, the phrase "under stringent conditions" includes, for example, methods described in Molecular Cloning - A LABORATORY MANUAL THIRD EDITION (Sambrook et al., Cold Spring Harbor Laboratory Press). For example, conditions for hybridization by performing incubation at 55°C or higher and 70°C or lower for several hours to overnight in a hybridization buffer consisting of 5 × SSC (composition of 20 × SSC: 3M sodium chloride, 0.3 M citric acid solution, pH 7.0), 0.1 % by mass of N-lauroyl sarcosine, 0.02% by mass of SDS, 2% by mass of blocking reagent for nucleic acid hybridization, and 50% formamide can be used. In addition, a washing buffer used at the time of washing after the incubation is preferably a 0.1% by mass SDS-containing 1 × SSC solution, and more preferably a 0.1% by mass SDS-containing 0.1 × SSC solution.

In addition, the base sequence of the nucleic acid may be codon-optimized for expression in a host (mainly mammalian animals, preferably human) In general, codon optimization refers to a process of modifying a nucleic acid sequence for enhanced expression in a host by replacing at least one codon of a native sequence with a codon used more frequently or most frequently in a gene of the host while maintaining a native amino acid sequence. Various species exhibit a specific bias for a specific codon of a specific amino acid. The codon bias (difference in codon usage frequency between organisms) is often correlated with the translation efficiency of mRNA, which is considered to depend particularly on the characteristics of the translated codon and the availability of the specific tRNA. The dominance of the selected tRNA in the cell generally reflects the most frequently used codon in peptide synthesis. Therefore, the gene can be individualized for optimal gene expression in a given organism based on codon optimization. The codon usage frequency table is easily available in, for example, "Codon Usage Database" published on www.kazusa.or.jp/codon/, and the codon can be optimized using these tables (Nakamura Y et al., "Codon usage tabulated from the international DNA sequence databases: status for the year 2000", Nucl Acids Res, vol.28, no.1, p292, 2000). A computer algorithm for codon optimization of a specific sequence for expression in a specific biological species is also available, for example, in Gene Forge (Aptagen, Inc.; Jacobus, PA). One or a plurality of codons in the base sequence of the nucleic acid correspond to the codon most frequently used for a specific amino acid in host to be introduced.

Among these, a nucleic acid in which only consecutive bases from the 935th to the 1885th from the 5' terminus are deleted in the base sequence represented by SEQ ID NO: 7 (TDP-43 mRNA spliced with intron 6 and added with a sequence after splicing), or a nucleic acid consisting of consecutive bases from the 112th to the 816th from the 5' terminus in the base sequence represented by SEQ ID NO: 7 (nucleic acid consisting of exons 2 to 5 of TDP-43 mRNA), that is, a nucleic acid consisting of a base sequence represented by SEQ ID NO: 4 or 5 is preferable.

### [TDP-43 binding motif]

From the viewpoint of expressing the modified TDP-43 protein mainly in cells in which the expression of TDP-43 is abnormal, it is preferable that the nucleic acid be added with a 3' untranslated region (3' UTR) including a TDP-43 binding motif. As a result, as shown in Examples described later, in normal cells, TDP-43 binds to a TDP-43 binding motif in the 3' UTR, intron 7 is spliced, and the cells become sensitive to nonsense-mediated mRNA decay (NMD), and thus the expression of the modified TDP-43 protein can be restricted. On the other hand, in a pathological cell in which the intranuclear TDP-43 is reduced, splicing of the intron 7 is inhibited, and expression of the TDP-43 modified body can be enhanced.

The base length of the 3' UTR is not particularly limited, but can be set to, for example, 100 bp or more and 3,000 bp or less, 200 bp or more and 2,000 bp or less, or 300 bp or more and 1,500 bp or less.

The TDP-43 binding motif preferably consists of a sequence in which thymine and guanine are alternately repeated three or more times in the sequence; the larger the number of times thymine and guanine are repeated, the larger the number of bound TDP-43 molecules, and is preferably 3 or more, more preferably 4 or more, further more preferably 5 or more, further more preferably 6 or more, further more preferably 8 or more, and particularly preferably 9 or more. On the other hand, an upper limit of the number of repetitions is not particularly limited, but for example, it can be set to 100 times or less, 50 times or less, 30 times or less, 20 times or less, and 10 times or less.

Specific examples of the TDP-43 binding motif consisting of a sequence in which thymine and guanine are alternately repeated three or more times include tgtgtgtgtgtg (SEQ ID NO: 8) and gtgtgtgt (SEQ ID NO: 9), but the TDP-43 binding motif is not limited thereto.

One TDP-43 binding motif may be included in the 3'UTR, or 2 or more TDP-43 binding motifs may be included, but preferably 2 or more TDP-43 binding motifs are included, more preferably 2 or more and 5 or less TDP-43 binding motifs are included, and further more preferably 2 or more and 3 or less TDP-43 binding motifs are included.

An insertion position of the TDP-43 binding motif in the 3' UTR can be appropriately set based on the sequence information of the 3' UTR, and for example, a preferable insertion position can be set using the RBPmap disclosed in Reference 1 (PazI et al., "RBPmap: a web server for mapping binding sites of RNA-binding proteins.", Nucleic Acids Res., Vol. 42, Web server issue, pp.W361-W367, 2014.).

For example, as a position where the TDP-43 binding motif is inserted in the 3' UTR consisting of the base sequence represented by SEQ ID NO: 10, in the base sequence represented by SEQ ID NO: 10, a region from the 150th to the 400th from the 5' terminus or a region from the 500th to the 600th from the 5' terminus is preferable, a region from the 180th to the 220th from the 5' terminus or a region from the 510th to the 550th from the 5' terminus is more preferable, and a region from the 190th to the 200th from the 5' terminus or a region from the 525th to the 535th from the 5' terminus is further more preferable.

Preferable examples of the nucleic acid encoding the modified TDP-43 protein to which the 3' untranslated region (3' UTR) containing the TDP-43 binding motif is added include a nucleic acid consisting of a base sequence represented by SEQ ID NO: 6.

The nucleic acid can be synthesized using a known method. Examples of the synthesis method include a genetic engineering technique, a chemical synthesis method, and the like. Examples of the synthesis method by a genetic engineering technique include an in vitro transcription synthesis method, a synthesis method using a vector, a synthesis method using a PCR cassette, and the like.

Examples of the chemical synthesis method include a phosphoroamidide method, an H-phosphonate method, and the like. In addition, examples of the chemical synthesis method include a method using a commercially available automatic nucleic acid synthesizer and the like.

### [Vector]

The nucleic acid may be in the form of a vector that expresses the nucleic acid. The vector expressing a nucleic acid can be prepared, for example, by inserting a nucleic acid encoding a modified TDP-43 protein into a commercially available vector.

The vector may be any vector as long as it can express a nucleic acid in a target cell.

The vector can include a promoter that controls the expression of a nucleic acid. In the vector, a sequence encoding a nucleic acid is functionally linked to a promoter.

The promoter is not particularly limited, but it is preferable to use a promoter that expresses a nucleic acid in a neural tissue-specific manner. Examples of such a promoter include a human synapsin 1 (Syn1) promoter and the like.

In addition to the sequence encoding a nucleic acid, the 3' UTR, and the promoter, the vector may contain, as desired, an enhancer, a poly A addition signal, a marker gene, a replication initiation point, a gene encoding a protein that binds to the replication initiation point and controls replication, and the like.

The term "marker gene" refers to a gene that enables sorting or selection of cells by introducing the marker gene into cells.

Specific examples of marker genes include drug-resistant genes, fluorescent protein genes, luminescent enzyme genes, color-developing enzyme genes, and the like. These may be used alone or in combination of two or more kinds thereof.

Specific examples of the drug-resistant gene include a puromycin-resistant gene, a neomycin-resistant gene, a tetracycline-resistant gene, a kanamycin-resistant gene, a zeocin-resistant gene, a hygromycin-resistant gene, a chloramphenicol-resistant gene, or the like.

Specific examples of the fluorescent protein genes include green fluorescent protein (GFP) genes, yellow fluorescent protein (YFP) genes, red fluorescent protein (RFP) genes, or the like. Specific examples of the luminescent enzyme genes include luciferase genes and the like. Specific examples of the color-developing enzyme genes include β-galactosidase genes, β-glucuronidase genes, alkaline phosphatase genes, or the like.

The kind of vector is not particularly limited, and a known expression vector can be used.

Examples of the expression vector include plasmid vectors, virus vectors, or the like.

The plasmid vector is not particularly limited as long as it is a plasmid vector that can be expressed in a target cell. For example, in the case of animal cells, a generally used plasmid vector can be used as a plasmid vector for expression in animal cells. Examples of plasmid vectors for expression in animal cells include, but are not limited to, pX459, pA1-11, pXT1, pRc/CMV, pRc/RSV, pcDNAIINeo, or the like.

Examples of virus vectors include retrovirus (including lentivirus) vectors, adenovirus vectors, adeno-associated virus vectors, Sendai virus vectors, herpesvirus vectors, vaccinia virus vectors, poxvirus vectors, poliovirus vectors, Sindbis virus vectors, rhabdovirus vectors, paramyxovirus vectors, orthomyxovirus vectors, or the like.

### <Reagent for introduction>

The aggregation inhibitor of TDP-43 of the present embodiment can further contain a reagent to promote the efficiency of introducing the modified TDP-43 protein or the nucleic acid encoding the modified TDP-43 protein into a target cell.

Examples of the reagent include atelocollagen; liposomes; ionic lipid such as Lipofectamine (registered trademark), lipofectin, transfectam (dioctadecylamidoglycyl spermine; DOGS), 1,2-dioleoyl-sn-glycero-3-phosphoethanolamine (DOPE), 1,2-dioleoyl-3-trimethylammonium-propane (DOTAP), didodecyldimethylammonium bromide (DDAB), DHDEAB (N,N-di-n-hexadecyl-N-methyl, N-(2-hydroxyethyl)ammonium bromide), polybrene, and poly(ethyleneimine) (PEI); and the like.

### <Subject to be administered>

The aggregation inhibitor of TDP-43 of the present embodiment is administered to a subject which has a TDP-43 gene. The administration method can be carried out by bringing the aggregation inhibitor of TDP-43 into contact with a subject. The administration may be in vivo or in vitro.

The subject is not particularly limited, and examples thereof include cells, tissues, organs, or the like of mammalian animals such as humans, monkeys, marmosets, mice, rats, guinea pigs, dogs, cats, rabbits, cows, horses, pigs, goats, and sheep.

### «Pharmaceutical composition»

The pharmaceutical composition of the present embodiment contains the aggregation inhibitor of TDP-43 as an active ingredient, and is used at preventing or treating TDP-43 proteinopathy.

According to the pharmaceutical composition of the present embodiment, as shown in Examples described later, it is possible to inhibit the aggregation of the TDP-43 protein in the cytoplasm and the liquid-liquid phase separation, thus providing a composition that is effective at preventing or treating TDP-43 proteinopathy.

In the present specification, the phrase "containing as an active ingredient" means containing the aggregation inhibitor of TDP-43 in a therapeutically effective amount. The phrase "therapeutically effective amount" referred to here means an amount of the aggregation inhibitor of TDP-43 or a combination of the aggregation inhibitor of TDP-43 and one or more active agents, which induces a biological or medical effect or response required by a doctor, a clinician, a veterinarian, a researcher, or another appropriate expert in the case of being administered according to a desired therapeutic measure. The preferred therapeutically effective amount is an amount that improves the symptoms of TDP-43 proteinopathy. In addition, the term "therapeutically effective amount" includes an amount effective for prevention, that is, an amount suitable for preventing a disease state.

TDP-43 proteinopathy is a general term for neurodegenerative diseases in which TDP-43 proteins aggregate and accumulate, and examples thereof include frontotemporal lobar degeneration (FTLD) and amyotrophic lateral sclerosis (ALS). FTLD and ALS include both a genetic mutation type (familial) and sporadic.

In addition, since the accumulation of the TDP-43 protein has been confirmed in neurodegenerative diseases such as Alzheimer's disease, Lewy body dementia, Down syndrome, hippocampal sclerosis, familial British dementia, Perry syndrome, Parkinson's disease, polyglutamine disease (for example, Huntington's disease, spinocerebellar degeneration type 3, and the like), myopathy (for example, sporadic inclusion body myositis, inclusion body myopathy, ocular pharyngeal muscular dystrophy, distal myopathy, myofibrillar myopathy, and the like), cortical basal ganglia degeneration, progressive supranuclear palsy, and argyrophilic grain disease, in addition to the accumulation of tau protein, amyloid β protein, and huntingtin protein, TDP-43 protein can also be applies to these diseases.

Among these TDP-43 proteinopathies, TDP-43 protein is suitably used for treatment or prevention of FTLD or ALS.

The pharmaceutical composition of the present embodiment may be formulated and used using the aggregation inhibitor of TDP-43 in an effective amount alone or in combination with a pharmaceutically acceptable carrier.

In the case where the aggregation inhibitor of TDP-43 contained in the pharmaceutical composition of the present embodiment is a nucleic acid, the aggregation inhibitor may be in the form of a nucleic acid molecule, may be in the form of a vector containing a nucleic acid as described above, or the forms may be mixed.

Examples of the pharmaceutically acceptable carrier include excipients such as sucrose or starch; binding agents such as cellulose or methyl cellulose; disintegrating agents such as starch or carboxymethyl cellulose; lubricants such as magnesium stearate or aerogel; aromatics such as citric acid or menthol; preservatives such as sodium benzoate or sodium hydrogen sulfite; stabilizers such as citric acid or sodium citrate; suspending agents such as methyl cellulose or polyvinylpyrrolidone; dispersing agents such as surfactants; diluents such as water or physiological saline; and base waxes, but the carrier is not limited thereto.

In addition, the pharmaceutical composition of the present embodiment may be a pharmaceutical composition in which the aggregation inhibitor of TDP-43 is enclosed in a liposome. The liposome is a microclosed vesicle having an inner phase surrounded by one or more lipid bilayers, and can generally retain a water-soluble substance in the inner phase and a fat-soluble substance in the lipid bilayer. The "enclosed" described herein includes a state in which the aggregation inhibitor of TDP-43 is retained in the inner phase of the liposome and a state in which the aggregation inhibitor of TDP-43 is retained in the lipid bilayer.

The liposome may be a monolayer membrane or a multilayer membrane. In addition, the particle diameter of the liposome is, for example, 10 nm or more and 1,000 nm or less, and preferably 50 nm or more and 300 nm or less. In consideration of the deliverability to a target cell or a target tissue, the particle diameter is more preferably 50 nm or more and 200 nm or less, and further more preferably 50 nm or more and 100 nm or less.

Examples of the method of enclosing the aggregation inhibitor of TDP-43 in liposomes include a lipid film method (vortex method), a reverse evaporation method, a surfactant removal method, a freeze-thaw method, and a remote loading method, but the method is not limited thereto, and any known method can be appropriately selected.

The pharmaceutical composition of the present embodiment can be administered to a mammalian animal orally or parenterally, but is preferably administered parenterally. As the mammalian animal, the same exemplary examples as those in the above-described "aggregation inhibitor of TDP-43" can be used.

Examples of the parenteral administration method include subcutaneous injection, intramuscular injection, local injection, intraperitoneal administration, and intrathecal administration.

Examples of the preparation suitable for parenteral administration include sterile isotonic aqueous and non-aqueous injection agents, and the injection agent may further contain an antioxidant, a buffer solution, a bactericidal agent, an isotonic agent, and the like. Alternatively, sterile aqueous and non-aqueous suspending agent may be used, and the injection agent may further contain a suspending agent, a solubilizing agent, a thickener, a stabilizer, a preservative, or the like.

These preparations can be enclosed in a container in unit doses or in multiple doses at a time, such as an ampoule or a vial. In addition, the active ingredient and the pharmaceutically acceptable carrier can also be freeze-dried and stored in a state where the active ingredient and the pharmaceutically acceptable carrier only need to be dissolved or suspended in an appropriate sterile vehicle immediately before use. Examples of another preparation suitable for parenteral administration include a spray agent.

The content of the aggregation inhibitor of TDP-43 in the pharmaceutical composition of the present embodiment is not particularly limited, but for example, it can be set to about 0.1% by mass or more and 100% by mass or less with respect to the total mass of the pharmaceutical composition.

The dose of the pharmaceutical composition of the present embodiment varies depending on the purpose of administration, the method of administration, the type and severity of the subject disease, or the condition (sex, age, weight, or the like) of the subject, but, for example, in the case of systemic administration to an adult, the dose of the aggregation inhibitor of TDP-43 as a protein can be set to 3.0 × 10-10 mol/kg or more and 5.0 × 10-7 mol/kg or less as a single dose of the protein in the case where the aggregation inhibitor of TDP-43 is a protein. In addition, for example, in the case of local administration to an adult, the dose can be set to 3.0 × 10-11 mol/kg or more and 5.0 × 10-8 mol/kg or less.

In the case of a nucleic acid, the single administration dose of the nucleic acid can be set to 1.0 × 10-13 mol/kg or more and 1.0 × 10-9 mol/kg or less. In addition, for example, in the case of local administration to an adult, the dose can be set to 1.0 × 10-14 mol/kg or more and 1.0 × 10-10 mol/kg or less.

Such an administration dose can be administered one or more times and 10 or less times. In addition, from the viewpoint of maintaining a high effect of the aggregation inhibitor of TDP-43, it is preferable that the TDP-43 be additionally administered at regular intervals. The administration interval is not particularly limited, and examples thereof include every day, every 3 days, every week, every 2 weeks, every month, every 3 months, and every 6 months.

The pharmaceutical composition of the present embodiment can be used in combination with, for example, a therapeutic drug for a TDP-43 proteinopathy such as FTLD and ALS, for example, a therapeutic drug for these diseases that have already been marketed. Examples of the therapeutic drug include a brain protective agent (for example, edaravone and the like), a glutamate action inhibitor (for example, riluzole and the like), or a neurotrophic factor (for example, insulin-like growth factor-1, or 5-HT1a receptor agonist (for example, Xaliproden) and the like). These combination drugs can be formulated together with the pharmaceutical composition of the present embodiment and administered as a single preparation, or can be formulated separately from the pharmaceutical composition of the present embodiment and administered at the same time or with a time lag in the same or a different administration method as the pharmaceutical composition of the present embodiment. In addition, the dose of these combination drugs may be an amount that is usually used in the case where the drug is administered alone, or the dose may be reduced from the amount that is usually used.

### <Treatment method>

In one embodiment, the present invention provides a method for preventing or treating a TDP-43 proteinopathy, the method including administering an effective amount of the aggregation inhibitor of TDP-43 to a patient or an affected animal in need of a medical treatment. Examples of the aggregation inhibitor of TDP-43 include the same ones as those described above. In addition, examples of the affected animal include the exemplary examples of the mammalian animals. In addition, examples of TDP-43 proteinopathy include the same ones as those described above, but among these, FTLD or ALS is preferable. That is, the method for preventing or treating TDP-43 proteinopathy can also be a method for preventing or treating FTLD or ALS.

In one embodiment, the present invention provides an aggregation inhibitor of TDP-43 at preventing or treating TDP-43 proteinopathy. Examples of the aggregation inhibitor of TDP-43 include the same ones as those described above. In addition, examples of TDP-43 proteinopathy include the same ones as those described above, but among these, FTLD or ALS is preferable.

In one embodiment, the present invention provides a use of the aggregation inhibitor of TDP-43 for producing a pharmaceutical composition used at preventing or treating TDP-43 proteinopathy. Examples of the aggregation inhibitor of TDP-43 include the same ones as those described above. In addition, examples of TDP-43 proteinopathy include the same ones as those described above, but among these, FTLD or ALS is preferable.

### Examples

Hereinafter, the present invention will be described below with reference to Examples, but is not limited to the following Examples.

### [Example 1]

### (Test to confirm liquid-liquid phase separation of TDP-43)

The present inventors hypothesized that in the case where a TDP-43 isoform (sTDP) in which an intron 6 of TDP-43 lacking an IDR is spliced binds to TDP-43 through NTD, the IDR density is inhibited, and as a result, abnormal liquid-liquid phase separation and aggregation are inhibited (refer to FIG. 1). Therefore, in order to verify the hypothesis, first, a test to confirm the liquid-liquid phase separation of TDP-43 was carried out using sTDP.

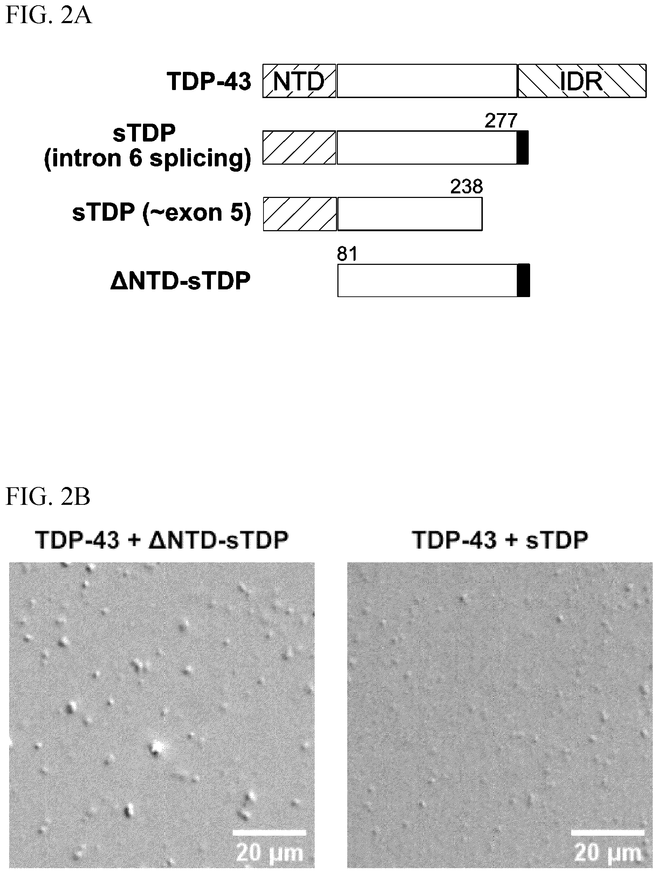

FIG. 2A is a schematic view showing TDP-43 and a modified body of TDP-43 used in Examples. In the following Examples, the following TDP-43 and modified bodies of TDP-43 were used. The modified bodies used in Examples 1 and 2 were prepared using an Escherichia coli expression system in which RosettaTM2 (DE3) pLysS Competent Cells (manufactured by Novagen, Inc.) were used as a host from a plasmid vector having a nucleic acid encoding a protein in which a Maltose-binding protein (MBP) tag was granted to each peptide represented by SEQ ID NOS: 1 to 3 or SEQ ID NO: 11. As the plasmid vector, pJ4M was used.

The amino acid sequence of TDP-43 is represented by SEQ ID NO: 1.

sTDP (intron 6 splicing) is a TDP-43 isoform in which intron 6 is spliced, and does not include amino acid residues from the 277th to the 414th from the N-terminus corresponding to the IDR, in the full-length amino acid sequence of TDP-43. The amino acid sequence of sTDP (intron 6 splicing) is represented by SEQ ID NO: 2. sTDP (to exon 5) consists of amino acid residues from the 1st to the 238th from the N-terminus, in the full-length amino acid sequence of TDP-43. The amino acid sequence of sTDP (to exon 5) is represented by SEQ ID NO: 3.

ΔNTD-sTDP does not include amino acid residues from the 1st to the 80th from the N-terminus corresponding to the NTD and amino acid residues from the 277th to the 414th from the N-terminus corresponding to the IDR, in the full-length amino acid sequence of TDP-43. The amino acid sequence of ΔNTD-sTDP is represented by SEQ ID NO: 11.

TDP-43 (5 µM) to which an MBP tag was added to the C-terminus was added to a solution (20 mM HEPES-NaOH (pH 7.4), 150 mM NaCl, 1 mM DTT) containing sTDP (intron 6 splicing) (5 µM) or ΔNTD-sTDP (5 µM), TEV protease (manufactured by New England Biolabs Inc.) was further added to cleave MBP, and then the solution was observed with a differential interference microscope after 30 minutes. FIG. 2B shows a differential interference microscope image.

As shown in FIG. 2B, it was observed that in the case where sTDP (intron 6 splicing) was mixed with TDP-43, the number and size of droplets exhibiting liquid-liquid phase separation were reduced as compared with the negative control in which ΔNTD-sTDP was mixed.

From the above results, it was found that sTDP (intron 6 splicing) inhibits abnormal liquid-liquid phase separation by TDP-43.

### [Example 2]

### (Test to confirm aggregation properties of TDP-43)

Next, a confirmation test was carried out on the aggregation properties of the modified body of TDP-43 itself and the aggregation inhibitory effect on TDP-43 due to the modified body of TDP-43.

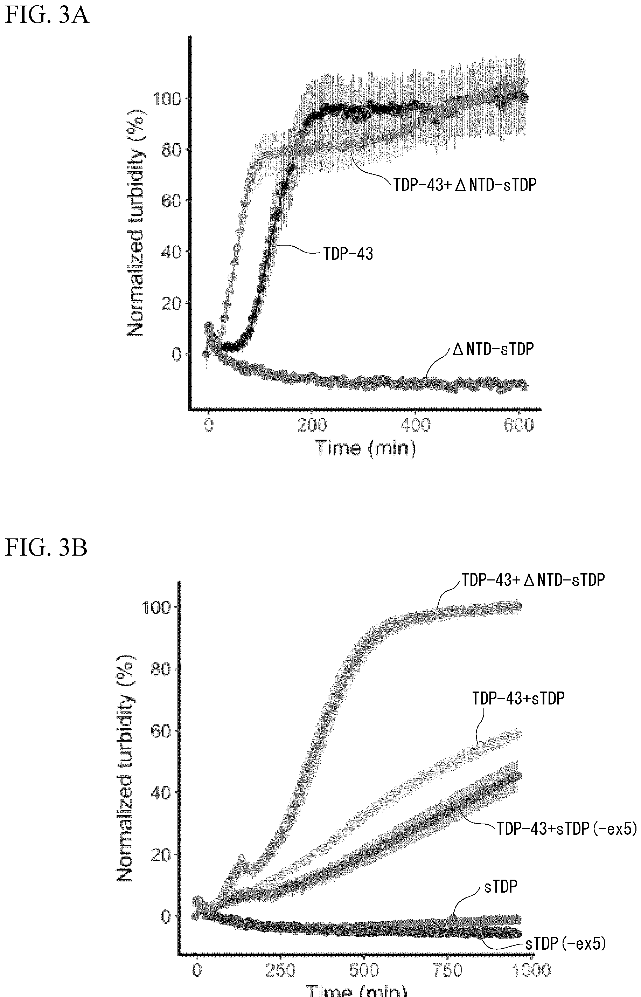

TEV protease (manufactured by New England Biolabs Inc.) was added to a solution (20 mM HEPEs-NaOH (pH 7.0), 150 mM NaCl, 1 mM DTT) containing TDP-43 (3 µM) to which an MBP tag was added to the C-terminus, ΔNTD-sTDP (3 µM), and a mixture thereof (each 3 µM), and MBP was cleaved, and then while shaking at 30°C using a TECANTM Filter Max (manufactured by Molecular Devices, LLC) plate reader, the relative turbidity represented by the absorbance at 405 nm was measured for 600 minutes. The results are shown in FIG. 3A.

As shown in FIG. 3A, ΔNTD-sTDP did not exhibit aggregation properties, but did not inhibit the aggregation of TDP-43.

Next, TEV protease (manufactured by New England Biolabs Inc.) was added to the following solution containing any one of 1) to 5) proteins to cleave MBP.
1) sTDP (intron 6 splicing) to which an MBP tag is added to the C-terminus (3 µM)
2) sTDP (to exon 5) to which an MBP tag is added to the C-terminus (3 µM)
3) TEV protease (manufactured by New England Biolabs Inc.) was added to a mixed protein (20 mM HEPES-NaOH (pH 7.0), 150 mM NaCl, 1 mM DTT) of TDP-43 to which an MBP tag was added to the C-terminus (3 µM) and ΔNTD-sTDP to which an MBP tag was added to the C-terminus (3 µM) to cleave MBP.
4) A mixed protein of TDP-43 to which an MBP tag is added to the C-terminus (3 µM) and sTDP (intron 6 splicing) to which an MBP tag is added to the C-terminus (3 µM)
5) A mixed protein of TDP-43 to which an MBP tag is added to the C-terminus (3 µM) and sTDP (to exon 5) to which an MBP tag is added to the C-terminus (3 µM)

Thereafter, while shaking at 30°C using a TECANTM Filter Max (manufactured by Molecular Devices, LLC) plate reader, the relative turbidity represented by an absorbance of 405 nm was measured for 1,000 minutes. The results are shown in FIG. 3B.

As shown in FIG. 3B, sTDP (intron 6 splicing) and sTDP (to exon 5) did not exhibit aggregation properties, and unlike ΔNTD-sTDP, inhibited the aggregation of TDP-43.

Therefore, since the aggregation inhibitory effect of TDP-43 was also recognized in sTDP (intron 6 splicing) as well as sTDP (to exon 5) in which the entire exon 6 was deleted, it was suggested that a modified body having an NTD of TDP-43 and lacking an IDR inhibits the aggregation of TDP-43.

### [Example 3]

### (Test to confirm stability of TDP-43)

Next, the stability of TDP-43 and a modified body of TDP-43 in a cell was confirmed.

First, a nucleic acid (SEQ ID NO: 12) encoding TDP-43 (TDP-43-Halo) to which a Halo tag was added to the C-terminus was inserted into a vector (manufactured by Vector Builder, Inc.) to construct a TDP-43-Halo expression vector. In addition, a nucleic acid (SEQ ID NO: 13) encoding sTDP (intron 6 splicing) (sTDP (intron 6 splicing)-Halo), in which a Halo tag was added to the C-terminus, was inserted into a vector (manufactured by Vector Builder, Inc.) to construct an sTDP (intron 6 splicing)-Halo expression vector.

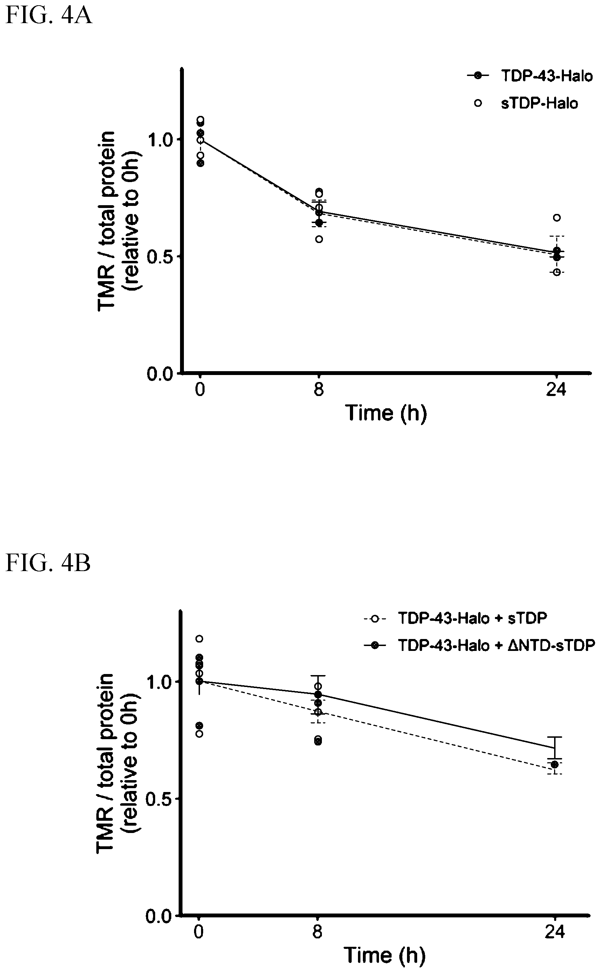

Next, a TDP-43-Halo expression vector (200 ng/well in 24 wells) or an sTDP (intron 6 splicing)-Halo expression vector (200 ng/well in 24 wells) was introduced into HEK293T cells, and a TMR ligand (G8252, manufactured by Promega Corporation) (100 nM) was added. After 40 minutes from the introduction, the culture medium was exchanged with a culture medium containing 7BRO (10 µM) that competes with the binding to the Halo tag, and a protein was extracted immediately after the culture medium exchange, 8 hours after the culture medium exchange, and 24 hours after the culture medium exchange. SDS PAGE was carried out, and a fluorescence value of the TMR ligand was measured. The results are shown in FIG. 4A. A value of a vertical axis of the graph of FIG. 4A is a relative value obtained by correcting the fluorescence value of the TMR ligand with the total protein amount of the cells immediately after the culture medium exchange, which is measured using a No stain Protein labeling Reagent (manufactured by Thermo Fisher Scientific, Inc.).

As shown in FIG. 4A, TDP-43 and sTDP (intron 6 splicing) showed the same expression pattern in cells.

Next, the influence of the presence of a modified body of TDP-43 on the expression level of TDP-43 was confirmed.

First, a vector (ΔNTD-sTDP-Halo expression vector) containing a nucleic acid (SEQ ID NO: 14) encoding ΔNTD-sTDP (ΔNTD-sTDP-Halo), in which a Halo tag was added to the C-terminus, was created by modifying a vector containing a nucleic acid encoding sTDP (intron 6 splicing)-Halo.

Next, a TDP-43-Halo expression vector (200 ng/well in 24 wells), and an sTDP (intron 6 splicing)-Halo expression vector (200 ng/well in 24 wells) or a ΔNTD-sTDP-Halo expression vector (200 ng/well in 24 wells) were introduced into HEK293T cells, and a TMR ligand (G8252, manufactured by Promega Corporation) (100 nM) was added. After 40 minutes from the introduction, the culture medium was exchanged with a culture medium containing 7BRO (10 µM) that competes with the binding to the Halo tag, and a protein was extracted immediately after the culture medium exchange, 8 hours after the culture medium exchange, and 24 hours after the culture medium exchange. SDS-PAGE was performed, and the fluorescence value of the TMR ligand was measured. The results are shown in FIG. 4B. The value of the vertical axis of the graph of FIG. 4B is a relative value obtained by correcting a fluorescence value of the TMR ligand with the total protein amount measured for the cells immediately after the culture medium exchange using a No stain Protein labeling Reagent (manufactured by Thermo Fisher Scientific, Inc.).

As shown in FIG. 4B, it was suggested that there was no difference in the stability of TDP-43 between the sTDP (intron 6 splicing) expression and the ΔNTD-sTDP expression, and the modified body of TDP-43 did not affect the expression level of TDP-43 itself.

### [Example 4]

### (In vivo test using human TDP-43 transgenic mouse)

A test of administering a modified body of TDP-43 to a human TDP-43 transgenic mouse was carried out.

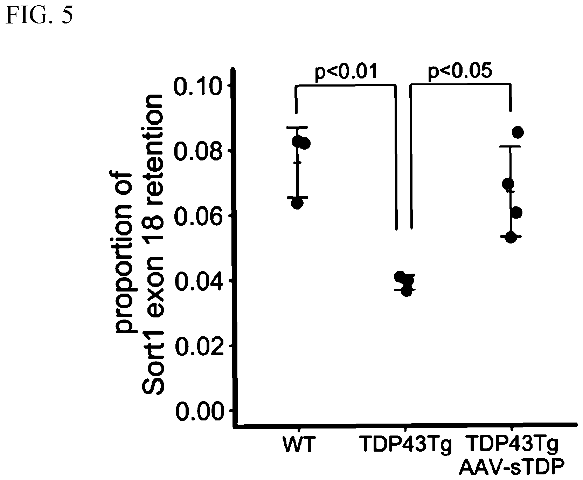

An adeno-associated virus vector (AAV-PHP.eB) expressing sTDP (intron 6 splicing) (base sequence: SEQ ID NO: 4) under a Syn1 promoter was administered to the ventricles of a human TDP-43 transgenic mouse (C57BLJ6-Tg (Prnp-TARDBP) 3cPtrc/J) at 1 day of age. After the mouse was aged 8 weeks or older, when the mouse is considered to be an adult, RNA was extracted from the cerebral cortex of the mouse using NucleoSpin RNA (manufactured by Takara Bio Inc.), and then cDNA was obtained by reverse transcription using PrimeScript RT Master Mix (manufactured by Takara Bio Inc.). The retention rate of exon 18 of Sort1 mRNA, which is one of the target selective splicing of TDP-43, was analyzed by liquid droplet digital PCR. The results are shown in FIG. 5.

As shown in FIG. 5, the retention rate of exon 18 of Sort1 mRNA was decreased in a non-administered hemizygous mouse (TDP43Tg, n = 3) as compared with a non-administered wild-type (WT, n = 3). On the other hand, in the hemizygous mouse (TDP43TgAAV-sTDP, n = 4) to which AAV-sTDP (intron6 splicing) was administered, the decrease in the retention rate of exon 18 was inhibited.

Therefore, in the case where sTDP (intron 6 splicing) was expressed in the central nervous system of the human TDP-43 transgenic mouse, the RNA splicing abnormality due to the excessive TDP-43 was inhibited. This abnormality is also one of splicing abnormalities caused by a TDP-43 mutation, which is one of the causes ofALS. Therefore, it was suggested that expression of sTDP (intron 6 splicing) has an effect of inhibiting the pathological state of TDP-43 proteinopathy including ALS.

### [Example 5]

### (A modification test of 3' UTR of sTDP (intron 6 splicing))

It was examined that a TDP-43 binding motif was inserted into a 3' UTR of a modified body of TDP-43 for the purpose of construction of a system of expressing the modified body of TDP-43, mainly in cells in which the expression of TDP-43 is abnormal.

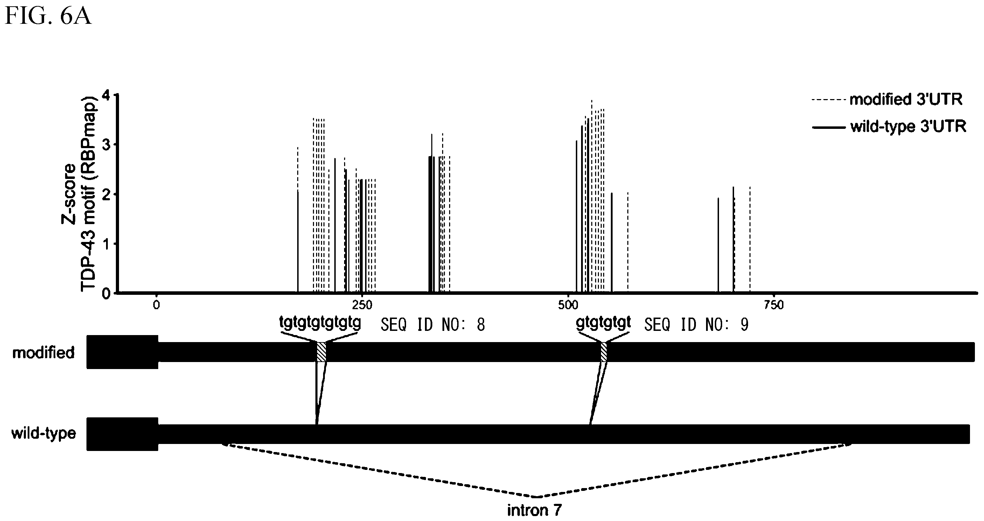

In FIG. 6A, the upper graph shows the position and strength (Z score) of the TDP-43 binding motif (gagug (SEQ ID NO: 15) or guaug (SEQ ID NO: 16)) in the wild-type 3'UTR of the sTDP (intron 6 splicing) and the position and strength (Z score) of the TDP-43 binding motif (tgtgtgtgtgtgtgtg (SEQ ID NO: 8) or gtgtgtgtgtgt (SEQ ID NO: 9)) inserted at two sites (between the 196th and the 197th and between the 529th and the 530th, of SEQ ID NO: 10) in the modified 3'UTR of the sTDP (intron 6 splicing), using RBPmap (Reference 1: Paz I et al., "RBPmap: a web server for mapping binding sites of RNA-binding proteins.", Nucleic Acids Res., Vol. 42, Web server issue, pp. W361-W367, 2014).

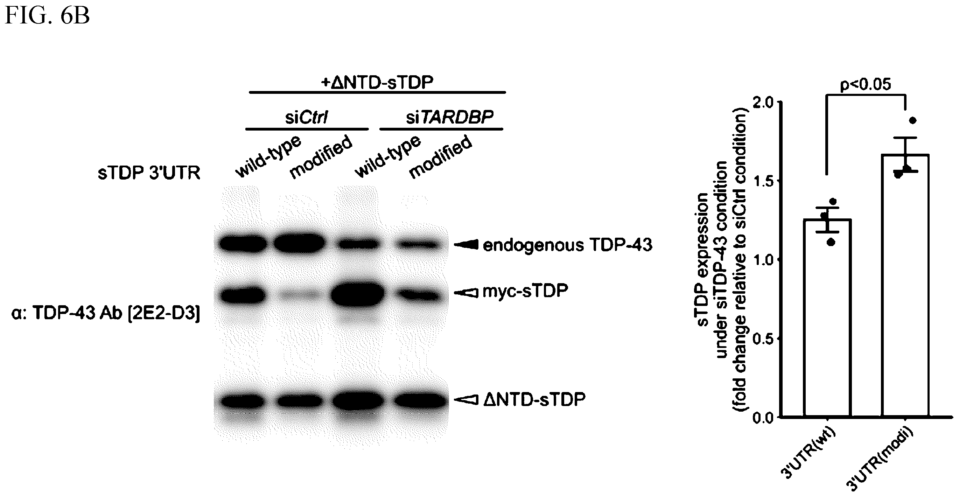

The myc-sTDP (intron 6 splicing)-3'UTR (wild-type) (hereinafter, referred to as "wild-type 3'UTR-added sTDP") (SEQ ID NO: 17) to which the wild-type 3'UTR was added, or the myc-sTDP (intron 6 splicing)-3'UTR (modified) (hereinafter, referred to as "modified 3'UTR-added sTDP") (SEQ ID NO: 18) in which the TDP-43 binding motif was inserted into two sites in the 3'UTR was transfected into HEK293T cells. The expression of sTDP (intron 6 splicing) was corrected using the expression of co-transfected ΔNTD-sTDP-myc (SEQ ID NO: 19). After 48 hours, the protein was extracted, and Western blotting was carried out using an anti-TDP-43 antibody (2E2-D3). The results are shown in FIG. 6B.

As shown in the analysis image of Western blotting on the left side of FIG. 6B, under normal conditions, it was observed that the expression of the modified 3'UTR-added sTDP is significantly inhibited as compared with the expression of the wild-type 3'UTR-added sTDP. In addition, under the condition in which TDP-43 is knocked down by the siRNA (SEQ ID NO: 20) targeting the inside of intron 6 of TDP-43 mRNA (Reference 2: Ishihara, et al., "Decreased number of Gemini of coiled bodies and U12 snRNA level in amyotrophic lateral sclerosis.", Human Molecular Genetics, Vol. 22, No. 20, pp. 4136-4147, 2013), it was observed that the expression of the modified 3'UTR-added sTDP is increased. This increase was more significant than the increase in the expression of the wild-type 3'UTR-added sTDP under the condition in which TDP-43 was knocked down (refer to left graph of FIG. 6B).

From the above results, it was clarified that by adding the 3'UTR, in which the TDP-43 binding motif was overlapped and inserted, to a code region of the modified body of TDP-43, the expression of the modified body of TDP-43 becomes acute by the expression level of the intranuclear TDP-43. Specifically, in the modified body of TDP-43 added with the 3'UTR, in which the TDP-43 binding motif was overlapped and inserted, in a normal cell, TDP-43 bound to the inside of 3'UTR, intron 7 was spliced, the cell became sensitive to NMD, and the expression was restricted. On the other hand, in the pathological cells in which the intranuclear TDP-43 was reduced, splicing of the intron 7 was inhibited, and the expression of the modified body of TDP-43 was enhanced. Therefore, it was confirmed that by adding 3'UTR, in which the TDP-43-binding motif was overlapped and inserted, to a code region of the modified body of TDP-43, the expression of the modified body of TDP-43 can be induced mainly in cells in which the abnormality of TDP-43 has occurred.

### [Example 6]

In TDP-43, an intrinsically disordered region (IDR) at the C-terminus is mainly involved in aggregation. Therefore, an object of the present Example was to verify the hypothesis that sTDP forms a heterodimer with TDP-43 through an N-terminus domain (NTD) to inhibit the proximity of the IDR and inhibit the aggregation of TDP-43 in cells.

A split-GFP system was used for the verification. This is a system in which GFP molecules are reconstructed to emit fluorescence in the case where the separated GFPs approach each other at a sufficiently close distance.

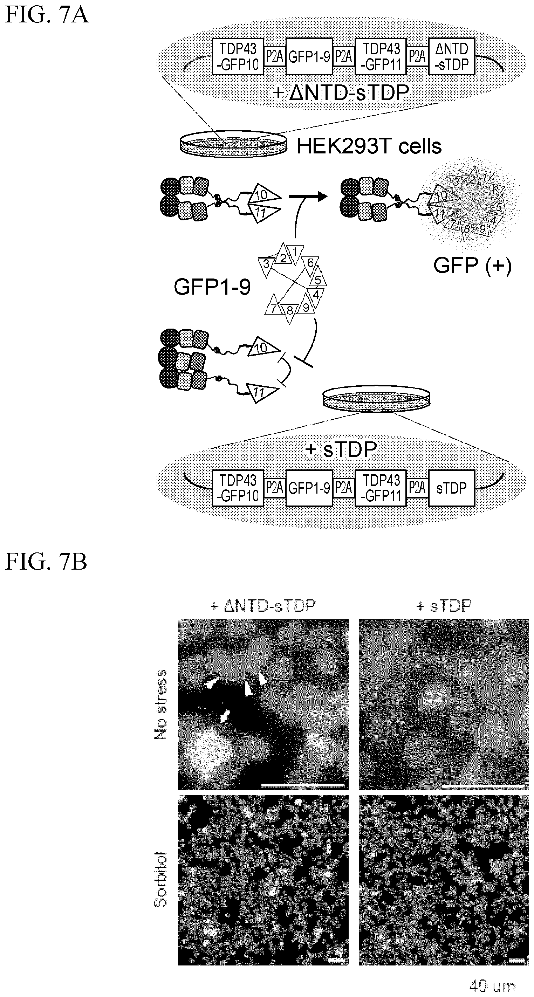

FIG. 7A shows a schematic view of the experimental system.

A plasmid vector, in which GFP10 or GFP11 was attached to the C-terminus of TDP-43 and which simultaneously expressed the remaining GFPs 1 to 9 and sTDP, was prepared. As a control, a plasmid vector expressing sTDP lacking NTD (ΔNTD-sTDP) was used instead of sTDP. These were transfected into HEK293T cells, and the fluorescence of GFP was observed.

In addition, in order to induce mislocalization of TDP-43 into the cytoplasm, the verification was carried out even under high osmotic pressure conditions in which Sorbitol was loaded in a culture medium at a final concentration of 0.4 M for 1 hour.

FIG. 7B shows a photomicrograph in which fluorescence of GFP was observed. The case where the addition was carried out using Sorbitol and the case where the load was not applied (No stress) are shown as both the case of ΔNTD-sTDP and the case of sTDP, of the control. The scale bar is 40 µm.

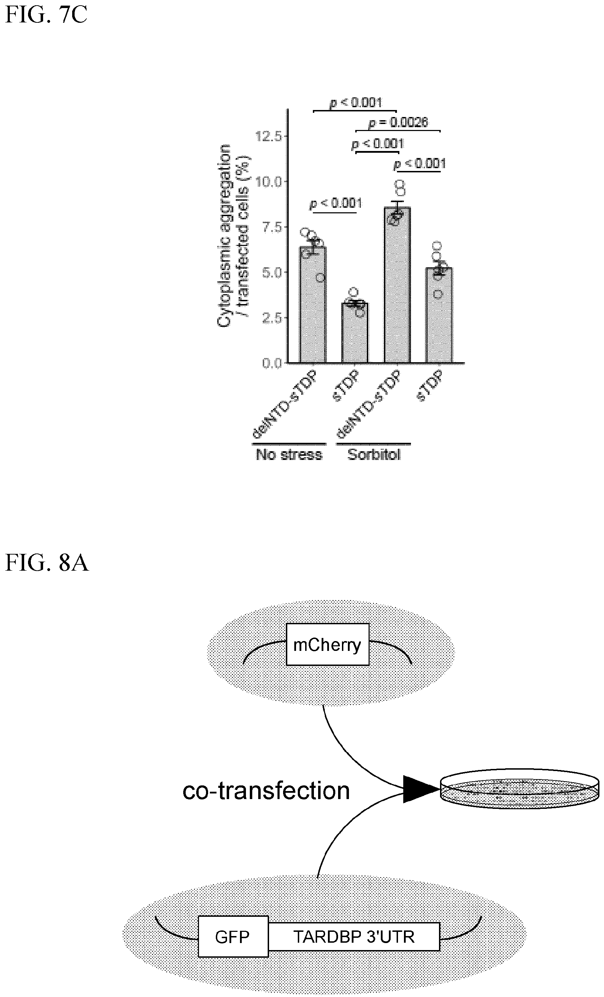

FIG. 7C shows the proportion of cells in which aggregates were recognized in each system.

The comparison was carried out by ANOVA and Tukey tests.

As shown in the figures, the cells expressing sTDP had fewer GFP granules in the cytoplasm than the cells expressing ΔNTD-sTDP (arrowheads in FIG. 7B). In addition, as shown in FIG. 7C, a proportion of cells in which large aggregates (arrow in FIG. 7B) were recognized in the cytoplasm was smaller in the sTDP expression group under both the non-stress condition and the Sorbitol loading condition.

From the above results, it was observed that sTDP inhibits the proximity of the IDR of TDP-43 in the cell and has an aggregation inhibitory effect.

### [Example 7]

Verification of a system for controlling sTDP expression by modifying TARDBP 3'UTR was carried out. The present embodiment may be a system in which the expression is induced in a pathological cell in which the intranuclear TDP-43 is reduced in a more general protein expression, not limited to the sTDP observed in the present specification. Therefore, a vector expressing a gene in which the 3'UTR of TARDBP was added to GFP was prepared, and this hypothesis was verified.

A vector expressing mCherry was co-expressed as a control of transfection.

FIG. 8A shows a schematic view of the experimental system.

FIG. 8B shows a photomicrograph in which fluorescence of GFP was observed. For each of mCherry and the control, in both of which only GFP was expressed, UTR modification of each of the wild-type, the modified1-UTR and the modified2-UTR to be described later was carried out, and for each, a normal state (siCtrl) and a TDP-43 knocked-down condition by siRNA (siTDP43) were observed. The scale bar is 100 µm.

As shown in the figure, it was observed that the expression of GFP to which the modified 3'UTR was added is inhibited in a normal state as compared with the expression of GFP to which the wild-type 3'UTR was added.

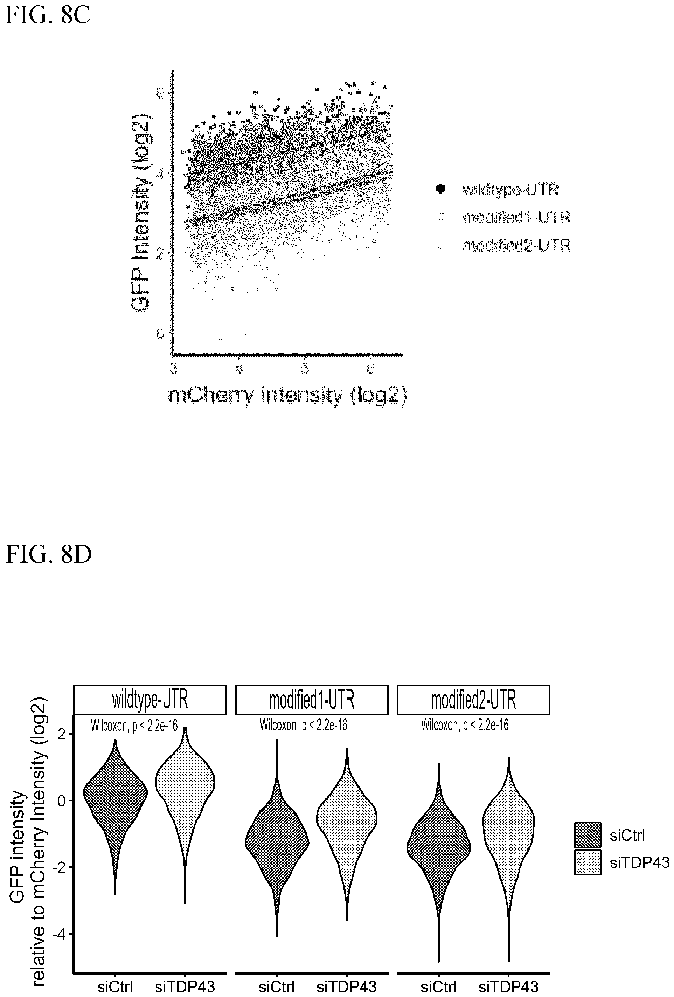

FIG. 8C shows the distribution of the correlation between the fluorescence intensity of GFP and mCherry for the wild-type, modified 1-UTR, and modified2-UTR.

The distribution was quantitatively determined by measuring a GFP fluorescence intensity for each cell expressing mCherry with respect to the inhibition in the modified UTR.

FIG. 8D shows a graph of the distribution of the correlation between GFP and mCherry for the wild-type, the modified1-UTR, and the modified2-UTR for each of siCtrl and siTDP43.

From FIGs. 8B and 8D, it was observed that the expression of GFP to which the modified 3'UTR was added was stronger in the TDP-43 knocked-down condition (siTDP43) by siRNA than in the normal state (siCtrl).

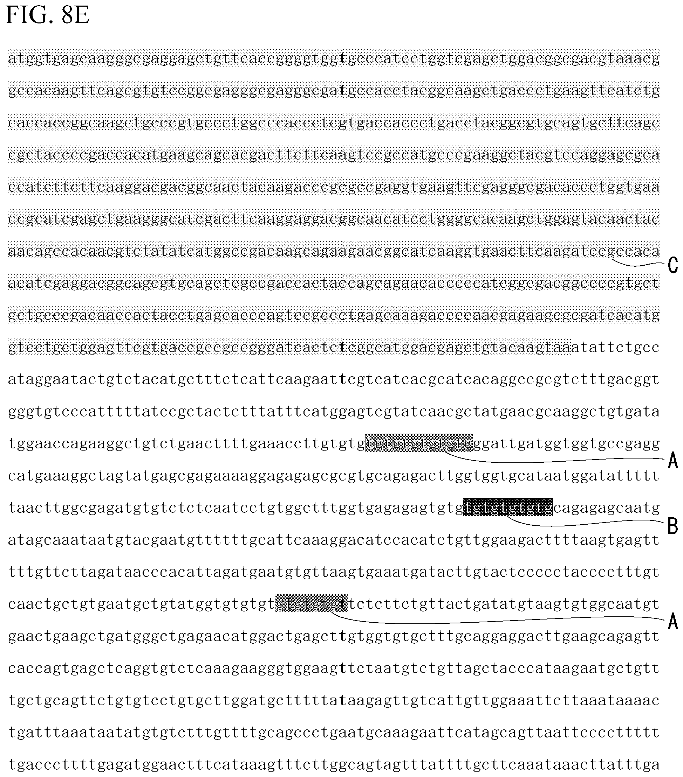

FIG. 8E shows modified portions of 3'UTR in the present experiment. The sequence shown in FIG. 8 is set as SEQ ID NO: 21.

In the modified1-UTR, modification of a portion of the figure having a dark shading (symbol A) is carried out. That is, as in Example 5, motifs repeating guanine and thymine corresponding to SEQ ID NO: 8 and SEQ ID NO: 9 were added at two positions.

The modified2-UTR has an arrangement of a site of inversion (symbol B) in addition to the site of the symbol A. That is, a motif in which guanine and thymine are repeated four times is further added.

In the figure, the light shading indicates a coding region of EGFP.

### INDUSTRIAL APPLICABILITY

The aggregation inhibitor of TDP-43 of the present embodiment acts specifically on TDP-43 and can inhibit the aggregation of TDP-43 without affecting the expression level of TDP-43 itself. The pharmaceutical composition of the present embodiment contains the aggregation inhibitor of TDP-43, and can prevent or treat TDP-43 proteinopathy.

## Claims

1. An aggregation inhibitor of TDP-43, comprising:
a modified TDP-43 protein which contains at least an N-terminus domain and does not contain an intrinsically disordered region at the C-terminus, or a nucleic acid encoding the modified TDP-43 protein.

2. The aggregation inhibitor of TDP-43 according to Claim 1, further comprising:
a protein consisting of an amino acid sequence which contains at least amino acid residues from the 1st to the 77th from an N-terminus and does not contain amino acid residues from the 278th to the 414th from the N-terminus in an amino acid sequence represented by SEQ ID NO: 1, or a nucleic acid encoding the protein.

3. The aggregation inhibitor of TDP-43 according to Claim 1 or 2, further comprising:
a protein consisting of an amino acid sequence represented by SEQ ID NO: 2 or 3, or a nucleic acid encoding the protein.

4. The aggregation inhibitor of TDP-43 according to Claim 1 or 2, further comprising:
a nucleic acid consisting of a base sequence represented by SEQ ID NO: 4 or 5.

5. The aggregation inhibitor of TDP-43 according to Claim 1 or 2,
wherein a 3' untranslated region including a TDP-43 binding motif is added to the nucleic acid.

6. The aggregation inhibitor of TDP-43 according to Claim 5,
wherein the TDP-43 binding motif consists of a sequence in which thymine and guanine are alternately repeated three or more times.

7. The aggregation inhibitor of TDP-43 according to Claim 6, further comprising:
a nucleic acid consisting of a base sequence represented by SEQ ID NO: 6.

8. A pharmaceutical composition comprising:
the aggregation inhibitor of TDP-43 according to Claim 1 or 2 as an active ingredient,
wherein the pharmaceutical composition is used at preventing or treating TDP-43 proteinopathy.

9. The pharmaceutical composition according to Claim 8,
wherein TDP-43 proteinopathy is frontotemporal lobar degeneration or amyotrophic lateral sclerosis.
